# EUROPEAN PATENT APPLICATION

(11) **EP 2 022 557 A1**
(43) Date of publication of application: **11.02.2009**
(21) Application number: 07405229.1
(22) Date of filing: 06.08.2007
(51) Int. Cl.: B01J 2/02, B01J 2/18, A61K 9/16

(54) **A process for the preparation of granular frozen powder**

(71) Applicant: Laboratories Development LLC, Miami, FL 33131 (US)
(72) Inventor: Vanalli, Marco, Scanzorosciate (BG) (IT)
(74) Representative: Biazzi, Riccardo

(57) **Abstract**

A process and method for the preparation of low moisture lyophilized powder, apparatus and methodology able to prepare powders solid forms using ultrasounds system and a spray cooling system and further lyophilization.

## Description

### TECHNICAL SECTOR

This invention applies, improving, the matter contained in the preparation technology of solid forms with controlled release of the components or more simply of ultrasound micro-encapsultaion contained in the patents:
IT n° 1274879
International patent n° PCT/IT/00048
CHINA patent n° ZL95190851.0
MEXICO patent n°192419
USA patent n° 5707636
EUROPE patent n° 726.765

And it has as object a methodology and a production system for the realisation of solid forms at controlled release of contained principles, forms obtained thanks to the use of nebulisers that work on the mechanical vibrations of sonodroti or of nozzles at ultrasound frequency.

This technology can be applied in the food, pharmaceutical, cosmetic, phitofarmaco, veterinary, health sectors, and in flavours, fragrances, enzymes, yeast and lactic and not lactic ferments and in fertilizers sector.

The new technology has like object a method and a production system for the realisation of solid, frozen, lyophilisable forms with characteristics such to give to every single particle a spherical form of dimensions that can vary from few micron (5-10) to a maximum of 800 micron.

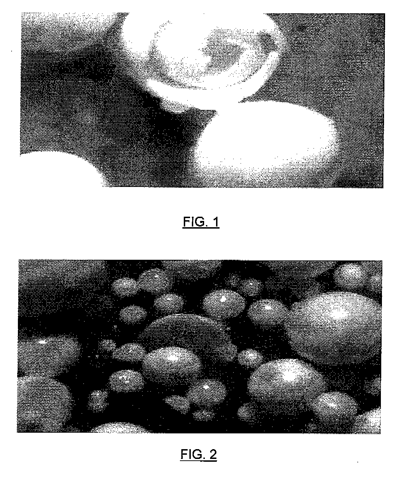

The deep study of characteristics of the products obtained trough ultrasounds systems above described and contained has allowed us to evidence that every single spherical particle obtained with this system, has like characteristic a multy layers composition (micro encapsulation), see attached Fig. 1.

This allows us to divide among them the different components of the processed products and, optimizing the cooling temperatures of the nebulisation chamber trough the use of cooling gas like liquid nebulised azotes, allows us a pre separation already in solid frozen form of the solvents (watery or not) from the solid ingredients that we want to produce; it is then known that the superficial tension of a drop of flat surface depends from the material and from temperatures, while if the drop is convex the superficial tension is inversely proportional to the ray of curving of the drop. So this technology can already separate in solid form the different components (micro encapsulation), besides it allows to prepare a frozen powder composed from very little micro spheres that so allow high speeds of drying processes or of lyophilisation.

### TECHNIQUE STATE

In this moment the lyophilizators are using frozen static techniques very slow that allow the forming of ice macro crystals that then, into the lyophilizer, need long time of sublimation.

In order to increase the efficiency of the lyophilizers they try to do some mechanical millings on the frozen products in blocks but during this operation that are possible risks of overheating the product and relative tear or contamination of the finished product.

This new technology instead permits a continuous production of a frozen powder in spherical particle that can be feeded directly into the lyophilizer increasing considerably the efficiency of the sublimation times that can be reduced till 1/3 of the normal normal time.

### DETAILED PRESENTATION OF THE INVENTION

The purpose of this invention is to offer a productive system and a production technology that allows the preparation of different frozen powders (with application fields in the food, pharmaceutical, cosmetic, veterinary, phitofarmaco, health sectors and in flavouring and fragrances sector, enzymes, yeast and lactic and not lactic ferments, and in fertilizers sector) ready to be feed into a lyophilizer, exceeding the disadvantages of the known technologies above indicated.

We have been able to improve the technology of preparation of solid forms with controlled release obtaining a production capacity for each ultrasound unit that can reach the 200 kg/h.

The centrifugal force developed from the ultrasounds unit on every drop of nebulised product allows us to separate, during the phase of gravitational fall of the micro spheres, the different components of treated product and, trough a narrow check-up of temperatures of nebulisation chamber, we are able to crystallize the different components of the product so that we can check the crystallization and distribute them into the micro sphere in function of their specific weight.

In this way we are able to isolate or send the crystals of the solvent (watery or not) on the external surface of the frozen micro sphere, this will facilitate considerably the phase of sublimation.

This operation is then able to give a greater stability to the solid product obtained because it will not solubilise in solvent during the sublimation phase because it will result physically separated from the solvent.

The lyophilised obtained products present then very particular characteristics because, if the solid content in the frozen powder is sufficient they keep also after lyophilisation ,the spherical form of the frozen product giving to the final powder the following positivities:
a) the product doesn't need granulation (as is instead for the lyophilized products with normal technologies), as it is obtained as a granular powder (See photo of SAME lyophilised powder in Fig. 2)
b) The typical spherical form obtained, avoid the lumping of the product and delays in strong way the absorption of the external humidity accentuating the stability of all those products sensible to the external humidity
c) The powder presents very strong solubility; this is due to the particular spherical form obtained
d) It is possible to stabilize the processed product in desired crystalline phases and of most stable form thanks to the many conditions of crystallization obtaining through the check-up of the frequencies of vibration of the ultrasounds units and to the cooling temperatures

Here attached some explicative examples

### Production of granular powder based on Nicorandil and process for their obtaining

### SUMMARY

We describe a process for the granular powder preparation of Nicorandil obtained with the technique of the spray cooling combined with the ultrasounds and further lyophilisation. The product obtained trough the process results stable and presents low humidity and hygroscopicity.

This invention is referred to a process for the preparation of Nicorandil obtained with the technique of spray cooling by ultrasounds and further lyophilisation. In particular it is referred to a process for the preparation of a granular spherical powder containing Nicoradil, particularly stable in order to be used as obtained in the different pharmaceutical preparations.

Nicoradil is a derivate from Niacinamide that works opening the channels of potassium ATP-dependents (K ATP) with a reduction either of pre-load or of the after-load, and improvement of coronary flux. Opening of channels of the potassium ATP-dependents gives to Nicoradil strong heart protection characteristics.

IONA study (Impact of Nicorandil in Angina; Lancet 2002; 359: 1269-1275) has evaluated the capacity of Nicoradil in reducing the frequency of coronary events in the patients with stable angina.

Patients with stable angina have been selected to receive Nicorandil (20 mg bid; n = 2.565) or a placebo (n = 2.561) in addition to the standard antiginous therapy.

The period of follow up has been 1,6 +/- 0,5 years. The primary, composite end point (mortality forcoronaropatia, not fatal myocardial infarct or hospitalization not previewed for thoracic pain of cardiac origin) has been reached in 13.1% in Nicorandil group and in 15.5% in the placebo group (HR 0.83, 95% Cl, 0.72-0.97; p=0,014).

Instead it has not been observed any differences in the secondary composite endpoint: 5.2% versus 4.2% respectively (HR 0.79, 95% Cl, 0.61-1.02; p = 0.068).

Coronary acute syndromes have been recognized in 7.6% of the patients of the placebo group and in 6.1% of the patients treated with Nicorandil (HR 0.79, 95%Cl, 0.64-0.98; p=0.028), while the total cardiovascular events have had an incidence of 17% versus 14.7%, respectively (HR 0.86, 95%Cl, 0.75-0.98; p=0.027).

IONA study has shown an important improvement of the outcome in patients with stable angina treated with Nicoradil. The efficiency and safety of Nicoradil have been compared with the one of the (ISMN) ones, in old patients (greater or equal to 65 years old) with stable angina pectoris and a positive proof under effort. 194 patients have been subjected in random way to a treatment with 10 mg of Nicorandil 2 times/day or to 20 mg of Isosorbide mononitrate 2 times/day.

In order to evaluate the antianginous efficacy, the patients have been subjected to a proof under effort after one week of treatment with placebo and after 4 weeks of treatment with Nicorandil or Isosorbide monoidrate. The safety and tolerance of two therapies has been evaluated every 2 weeks.

The study has demonstrated that, in spite of Nicorandil appears sure and effective like the isosorbide during the angina pectoris treatment in old patients, the isosorbide mononitrate appears superior to the Nicorandil during the symptomatic treatment of angina in this group of patients(Ciampiricotti R et al ,Am Heart j 2000; 139:e3).

In the United Kingdom, the angina hits about 10% of men after 60 years old, and it is a cause often under considered of heart diseases. Aspirin and ACE-inhibitors reduce the risk in the patients with stable angina, but we don't know the effects of anti-angina ones. An equip of researchers of Western Infirmary of Glasgow have studied 5126 patients randomized among 20 mg of Nicorandil (2 times day) or placebo further to the standard therapy (based on calcium antagonist or nitrates). 15.5% of placebo group and the 13.2% of the treated group has been hit from coronary death, not fatal infarct or new angina attack. The rate of acute coronary syndrome has been respectively 7.6% and 6.1%, while the rate of cardiovascular events has been 17% and 14.7% (Lancet, 13 apr. 2002, pages 1269).

So this substance has a very important pharmacological rule and it is normally used in hospital like heart protector it is yet known the difficult to use Nicorancidil like medicine because it appears extremely unstable at temperatures superior to 37°C and in presence of humidity.

The data indicated in literature (European patent EPA 0185347 A3) underline the following:
- Nicorandil stability depends from different factors, like the temperature, the humidity, the light and the oxygen;
- In order to prevent the degradation it is used the Nicorandil crystalline covered with hydrophobic materials like stearic alcohol;
- Even if it is relatively stable in its crystalline form, if it goes under compression, Nicorandil shows a reduction of the title;
- Adding of acids or saturated aliphatic alcohol (solids at room temperature) and organic acids like fumaric acid, ossalic, salicylic, tartaric or glutaric permits to obtain a solid formulation with improved stability.

Solid oral preparations containing Nicorandil result unstable in presence of humidity (cfr. H. Nagai et al., Chem.Pharm.Bull., 32, 1063, 1984); the same authors has noticed that the speed of degradation drastically reduce going from pH 11.0 to pH 4.0.

This observation has strengthened how indicated in literature (cfr. European Patent EP 0 185 347 A3 EP 230,932) about the stabilization of formulations of Nicorandil tablets realized with organic acids like fumaric, ossalic,silicylic etc. or formulates containing sugars with dimensions not superior to 10 pm and/or with organic acids.

The use of malic acid USP or other acids acceptable in pharmacy are an essential condition for the realisation of solid oral stable formulations.

The Italian patent n. EP 1001773 W09907370 describes a process for the preparation of acceptable pharmaceutical tablets of Nicorandil with modulated cession, characterized from a content of esters of glycerine with aliphatic fatty acids with number of carbon atoms equal or superior to 18.

These thing guarantees, once mixed with other pharmaceutical excipients purposely selected a certain stability of the final product.

Nevertheless, the resulting tablets are not more stable of the competition (IKOREL, ADANCOR) preserved in the same conditions.

In fact the experimental trials that want to characterize a packaging system suitable to guarantee the integrity of the product (protection from humidity) have confirmed that the system of packaging in blister aluminium/aluminium + humidity absorber, used for Ikorel^{®} and Adancor^{®} is the only able to guarantee a good stability to the tablets. So, it is normal to think that also for the formulations of the patents above indicated it is indispensable a packaging system identical in order to have a stability and so an identical validity period.

The most important factor of the instability of Nicorandil in every pharmaceutical compound today available is the internal humidity of the solid preparations, that in combination with temperatures of the product (25 - 40°C), degrade the molecule.

Infact is rather difficult to reduce the internal humidity to a very low level.

It is known that lower is water residue in the matter, higher is the stability of the product.

It is also known how the degradation speed is fastened by the storage temperature of the product.

This means that higher is the storage temperature of the product, faster will be the degradation of the product.

Formulations based on Nicorandil, if not formulated according to particular procedures and with specific attention, they reflect the above-mentioned instability of the active principle, (conversion in nicotinic acid and -N-(2-Idossietil)-nicotinammide), with evident negative repercussions on the preservation and store of the product, also for limited time periods.

Till today we don't know methods to stabilize the Nicorandil in definitive way or within acceptable percentages in oral solid formulations, in particular tablets avoiding the packaging according to the patent Shugai (Ikorel® ed Adancor®).

The only known concept is the necessity to keep under narrow control the humidity of the raw material protecting the tablets during compression phase and if possible and eventually follow the production step with a drying one.

So, today there is the necessity to find a simple and cheap process that allows obtaining a product based on Nicorandil, with low humidity content and protected from rehydration, in this way it is possible to increases stability in terms of active principle in favour of a stabilization of the active.

It has been wonderfully found that the obtaining of Nicorandil by ultrasonic spray cooling technique and further lyophilisation determines a better stability of Nicorandil raw material either due to the lowering of water content in the preparation obtained and for the reduced hydroscopicity of the powder obtained with this technology, as well for the form perfectly spherical of particles that are produced. It has further been observed the use of Nicorandil obtained with technique of ultrasound spraycooling and further lyophilisation instead than the one produced with traditional technology has given more stable final products

The principal reason is due to the relative higher hydroscopicity of the powder of traditional Nicorandil as regards to the granular powder obtained with ultrasonic spray cooling.

This involves that mixing the Nicorandil with excipients at variable humidity to form a pharmaceutical preparation its stability is depending from his hydroscopicity.

For this reason till today, in a direct mix and/or preparations of Nicorandil with other exipients, it has never been possible to have a stability of the active principle and so, a reduced degradation speed.

In table 1 are indicated the values of humidity of five lots of traditional raw material of Ncorandil with relative analytical characterization with store at 20°C for 30 days and of relative accellerated stability at 45°C for 90 days stored into closed containers.

| **Table 1:** | | | | | | | | | |
|---|---|---|---|---|---|---|---|---|---|
| ***stress test 90 days at 45 °C*** | | | | | | | | | |
| lots | Humidity% K.F. t=0 | Humidity % K.F. t= 30days at 20 °C | Nicorandil title % t=0 | Nicotinic acid % | Total impurities %t=0 | ***Humidity % K.F.*** | ***Nicorandil title %*** | Nicotinic acid% | ***Total impurities%*** |
| 001 | 0.45 | 0.46 | 99.66 | 0.18 | 0.36 | 0.46 | 96.76 | 0.38 | ***1.17*** |
| 002 | 0.68 | 0.60 | 99.78 | 0.14 | 0.33 | 0.68 | 96.75 | 0.34 | ***1.54*** |
| 003 | 0.66 | 0.65 | 99.76 | 0.17 | 0.33 | 0.65 | 96.78 | 0.37 | ***1.55*** |
| 004 | 0.59 | 0.56 | 99.43 | 0.16 | 0.34 | 0.55 | 95.99 | 0.46 | ***1.34*** |
| 005 | 0.54 | 0.54 | 99.49 | 0.16 | 0.44 | 0.50 | 96.19 | 0.36 | ***1.42*** |

In table 2 are indicated the values of humidity of the same five lots of raw material of Nicorandil re-solubilised in water and further processed by the technique of ultrasonic spray cooling and further lyophilisation always followed by storage at 20°C for 30 days and relative accelerated stability at 45°C for 90 days preserved into closed containers.

| **Table 2:** | | | | | | | | | |
|---|---|---|---|---|---|---|---|---|---|
| ***stress test 90 days at 45 °C*** | | | | | | | | | |
| lots | Humidity% K.F. t=0 | Humidity % K.F. t= 30 days at 20 °C | Nicorandil title % t=0 | Nicotinic acid % | Total impurities% t=0 | ***Humidity* % *K.F.*** | ***Nicorandil title %*** | Nicotinic acid% | ***Total impurities%*** |
| 001 | 0.24 | 0.23 | 99.61 | 0.15 | 0.34 | 0.16 | 98.66 | 0.28 | ***0.57*** |
| 002 | 0.34 | 0.33 | 99.45 | 0.15 | 0.36 | 0.18 | 98.75 | 0.24 | ***0.44*** |
| 003 | 0.32 | 0.32 | 99.54 | 0.17 | 0.35 | 0.15 | 98.68 | 0.17 | ***0.55*** |
| 004 | 0.36 | 0.34 | 99.65 | 0.16 | 0.37 | 0.15 | 98.99 | 0.26 | ***0.54*** |
| 005 | 0.22 | 0.24 | 99.54 | 0.15 | 0.46 | 0.10 | 98.69 | 0.19 | ***0.62*** |

In table 3 are indicated the values of humidity of five lots of traditional raw material of Nicorandil mixed with 200% of microcrystalline cellulose with relative analytical characterization with storage at 20°C for 30 days and relative accelerated stability at 45°C for 90 days preserved into closed containers.

| **Table 3:** | | | | | | | | | |
|---|---|---|---|---|---|---|---|---|---|
| ***stress test 90 days at 45 °C*** | | | | | | | | | |
| lots | Humidity% K.F. t=0 | Humidity % K.F. t= 30 days at 20 °C | Nicorandil title % t=0 | Nicotinic acid % | Total impurities% t=0 | ***Humidity % K.F.*** | ***Nicorandil title %*** | Nicotinic acid% | ***Total impurities%*** |
| 001 | 1.46 | 1.47 | 99.54 | 0.17 | 0.38 | 1.43 | 85.66 | 6.28 | ***19.57*** |
| 002 | 1.86 | 1.98 | 99.56 | 0.15 | 0.39 | 1.86 | 84.75 | 5.24 | ***18.44*** |
| 003 | 1.69 | 1.77 | 99.55 4 | 0.16 | 0.39 | 1.62 | 87.68 | 5.17 | ***17.55*** |
| 004 | 1.81 | 1.76 | 99.76 | 0.19 | 0.36 | 1.84 | 86.99 | 5.26 | ***18.54*** |
| 005 | 1.63 | 1.87 | 99.50 | 0.19 | 0.45 | 1.65 | 85.69 | 5.19 | ***17.62*** |

In table 4 are indicated the values of humidity of the same five lots of raw material of Nicorandil reobtained re- solubilised in water and further processed by the technique of ultrasonic spray cooling and further lyophilisation mixed with 200% of microcrystalline cellulose with relative analytical characterization always after store at 20°C for 30 days and relative accelerated stability at 45°C for 90 days preserved into closed containers.

| **Table 4:** | | | | | | | | | |
|---|---|---|---|---|---|---|---|---|---|
| ***stress test 90 days at 45 °C*** | | | | | | | | | |
| lots | Humidity% K.F. t=0 | Humidity % K.F. t= 30 days at 20 °C | Nicorandil title % t=0 | Nicotinic acid % | Total impurities% t=0 | ***Humidity % K.F.*** | ***Nicorandil title %*** | Nicotinic acid% | ***Total impurities%*** |
| 001 | 0.45 | 0.47 | 99.64 | 0.17 | 0.36 | 0.23 | 96.76 | 3.28 | ***9.55*** |
| 002 | 0.86 | 0.88 | 99.56 | 0.17 | 0.39 | 0.26 | 94.85 | 2.14 | ***8.65*** |
| 003 | 0.70 | 0.77 | 99.56 | 0.19 | 0.38 | 0.32 | 97.48 | 2.27 | ***7.43*** |
| 004 | 0.81 | 0.73 | 99.79 | 0.19 | 0.36 | 0.34 | 96.59 | 3.36 | ***8.78*** |
| 005 | 0.71 | 0.84 | 99.58 | 0.16 | 0.43 | 0.35 | 95.69 | 2.39 | ***7.61*** |

From data indicated in tables 1, 2, 3, 4, it is evicted how the raw material Nicorandil obtained with the ultrasonic spray cooling system and further lyophilisation is more stable of the traditional product at 45°C for 90 days.

So, an object of this invention is compounds that include Nicorandil obtained with the technique of spray cooling to the ultrasounds and further lyophilisation.

Nicorandil compounds either in traditional powder or powder obtained according to the method of the following patent can be in form of direct mix, tablet, capsule, grain and/or powder.

In this invention, for direct mix we mean a powder mix of Nicorandil, in association only with excipients pharmaceutically acceptable.

The compositions in further examples are in tablet form, preferably simple tablet. The compositions using Nicorandil according to this invention result with a moisture that is about 60% less than the standard one about the 60% (KF) and result about 8 times less hygroscopic as indicated in the here under table 5.

**Table 5**

| Known tablets based on traditional Nicorandil tablet 20 mg | Known tablets based on traditional Nicorandil tablet 20 mg | Tablets with Nicorandil spraycooling (example 1) | Tablets with Nicorandil spraycooling (example 1) |
|---|---|---|---|
| KF% T=0 | KF% T=24h* | KF% T=0 | KF% T=24h* |
| Lot 01 1.02 | 0.1.00 | 0.35 | 0.36 |
| Lot 02 1.02 | 0.87 | 0.37 | 0.38 |
| Lot 03 1.01 | 0.98 | 0.44 | 0.49 |
| Lot 04 0.99 | 0.85 | 0.35 | 0.37 |
| Lot 05 0.98 | 0.86 | 0.23 | 0.29 |

| | | | |
|---|---|---|---|
| ● at 40°C, -75Rh KF (humidity according to Karl Fisher method) ● T = time | | | |

Another further object of the present invention is a method for stabilize the Nicorandil as regards USA patent 5707636

### EXAMPLES

### EXAMPLE 1

Tablets of 20 mg Nicorandil/tablet
Composition based on Nicorandil

| | |
|---|---|
| A. Nicorandil granular powder as regards patent | 20,00 mg |
| B Mg sulphate | 2,00 mg |
| C. Citric acid | 4,00 mg |
| D. Xylitol | 100, 00 mg |
| E. Mannitol | 80,00 mg |
| F. Mg stearate | 2,00 mg |
| G. Cellulose microcrystalline | 40,00 mg |
| H. Stearic acid | 5,00 mg |
| Total weight nucleus | 253,00 mg |
| I. Idrossipropilmetilcellulose | 5,00 mg |
| L. Titanium bioxide | 0,5 mg |
| M. Talc | 1,00 mg |
| N. Trietilcitrate | 0,50 mg |
| Total weight tablet | 260,00 mg |

### 1.1. Mixing

We move A, B, C, D, E, H and G, and the 50% of F, in the quantities above indicated, in the mixer, keep under agitation for about 30 minutes. At the end of this operation, we move the resulting mix in dry containers, always checking humidity and temperature.

### 1.2. Precompression

We proceed to the precompression of the mix through rotary machine equipped with forming cylinder of 25.0 mm. The hardness of the tablets produced, has to be regulated in order to produce subsequently a final product with good reologic characteristics.

### 1.3 Granulation

The tablets produced during the first working phase are granulated on net from 1000-1500 µm always in ambient with controlled humidity.

### 1.4 Mixing

We transfer the granulate obtained in phase 1.3 in the mixer, adding Mg stearate and keeping all under strong agitation for about 30 minutes. At the end of this operation, we transfer the mix obtained in dry containers.

### 1.5 Compression

We proceed with final compression of the granulate through rotary machine provided with forming cylinder of 8.00 mm regulating the weight to 253 mg/tablet and the compression force to al least 20 KP. The produced tablets have a hardness between 20 and 30 KP.

Friability: ≤ 1.0%; desegregation time: ≤ 15 minutes (measured as regards to the method described in U.S.P. XXIV ed.)
Humidity as regards K.F. ≤ 1.50%
Stability proofs on the tablets have been made to 40°C and 75% R.H. for six months. Samples have been preserved in blister al/al

**Table 6**

| Lot 001-tablet of 20 mg ion/tablet (composition qualitative/quantitative see example 1) | | | | | |
|---|---|---|---|---|---|
| Lot (T/t)¹ | Humidity % K.F. t=0 | Niorandil ⁵ title % | Nicotinic acid % ² | N-(2-Idrossiatil) ³ nicotinamide % | Total impurities % ⁴ |
| 001 (20/0) | 0,25 | 99,32 | 0,05 | 0,12 | 0,17 |
| 001A (40/1) | 0,32 | 99,30 | 0,12 | 0,21 | 0,33 |
| | | | | | |
| 001B (40/3) | 0,30 | 99,2 | 0,25 | 0,78 | 1,03 |
| | | | | | |
| 001C (40/6) | 0,24 | 98,9 | 0,38 | 1,18 | 1,56 |

| | | | | | |
|---|---|---|---|---|---|
| ¹Temperature (°C)/time (months); ²Nicotinic acid; ³N-(2-Idrossietil)nicotinamide, Total impurities; ⁴ Nicorandil (mg/tablet)⁵ Data in table 9 show the optimum stability of tablets. | | | | | |

### EXAMPLE 2

Tablets of 20 mg nicorandil/tablet
Composition based on Nicorandil

| | |
|---|---|
| A. Nicorandil traditional powder | 20,00 mg |
| B Mg Sulphate | 2,00 mg |
| C. Citric acid | 4,00 mg |
| D. Xilitol | 100,00 mg |
| E. Mannitol | 80,00 mg |
| F. Mg stearate | 2,00 mg |
| G. Cellulose microcrystalline | 40,00 mg |
| H. Stearic acid | 5,00 mg |
| Total weight nucleus | 253,00 mg |
| I.Idrossipropilmetilcellulose | 5,00 mg |
| L. Titanium bioxide | 0,5 mg |
| M. Talc | 1,00 mg |
| N. Trietilcitrate | 0,50 mg |
| Total weight tablet | 260,00 mg |

Tablets have been obtained as regards to the processes of example 1 Stability proofs on the tablets have been made to 40°C and 75% R.H. for six months. Samples have been preserved in blister al/al

**Table 7**

| Lot 001-nucleuses of 20 mg ion/tablet (composition qualitative/quantitative see example 1) Lot 001-nucleuses da 20 mg ion/tablet (composition qualitative/quantitative see example 1) | | | | | |
|---|---|---|---|---|---|
| Lot (Tit)¹ | Humidity % K.F. t=0 | Niorandil ⁵ title % | Nicotinic acid % ² | N-(2-Idrossietil) ³ nicotinamide % | Total impurities % ⁴ |
| 001 (20/0) | 0,76 | 99,65 | 0,07 | 0,17 | 0,24 |
| 001A (40/1) | 0,87 | 95,30 | 0,72 | 0,67 | 1,39 |
| | | | | | |
| 001B (40/3) | 0,90 | 89,2 | 2,25 | 1,78 | 4,03 |
| | | | | | |
| 001C (40/6) | 0,88 | 78,9 | 4,38 | 3,18 | 7,56 |

| | | | | | |
|---|---|---|---|---|---|
| ¹Temperature (°C)/time (months); ²Nicotinic acid; ³N-(2-Idrossietil)nicotinamide, Total impurities; ⁴ Nicorandil (mg/tablet)⁵ | | | | | |

Data of table 9 subscribed K.F. higher and so, degradations much more evident through the formulation of example 1.

### Example 2

### LYOPHILISED COFFEE

We have worked on a liquid express coffee preparation to 30% of solids contents a part of product has been processed into our system obtaining a frozen, microencapsulated product and one part has been processed with a classic system; the products then have been lyophilised with the same curves of temperature of sublimination subscribing a time of 78 hours for the standard product and of 32 hours for the prepared product with our technology.

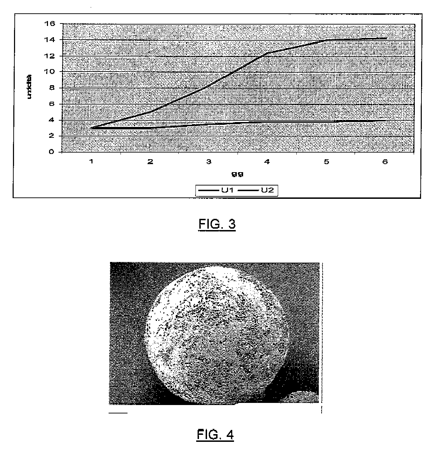

We have also noticed the following differences of absorption of the humidity from the ambient of these two samples, U1 corresponds to the product treated with our system, U2 is the product obtained with normal technology of lyophilisation; it is clearly visible a big difference in stability of the finished product (see Fig. 3).

The same type of result has been obtained treating some milk powder and some strawberry juice.

Fig. 4 shows milk powder lyophilised with new technology.

Since the examples are supplied for illustrative purposes, they have not to be absolutely considered limitative of this invention and besides it is easily understandable that anyone expert in these sectors could give other products or exemplifying substances that fully enter in the invention field how above described and so they cannot be considered uprooted of the claims under indicated.

## Claims

1. Methodology and equipments for the preparation of solid frozen and not frozen forms with controlled release of the solvents (watery and not) contained in treated products suitable to the lyophilisation with production of products that result more stable to shelf-life.

2. The system includes an ultrasounds nebulisation apparatus that nebulises in very small drops the treated products, the products can be compounds or simple and they can contain one or more solvents polar or non polar or material for fusion (fats / waxes) (see patents indicated to pag.1).

3. A cylindrical cooling chamber with vertical axis that allows the crystallization of the products that form the mix fed in the nebuliser.

4. The nebuliser at point 2, uses ultrasonic frequencies included in the range from 20 to 150 kHz, equipped of metallic sonodroti with appropriate shape depending of the treated products like, for example, semi spherical sonodroti.

5. The micro spheres produced have a size that goes from 5 to 800 micron.

6. The feeding system of the products on the sonodroto, is termoregulated and is feeded by gravity system.

7. Solidification temperature control of the micro spheres through nebulisation of liquid refrigerating gas in order to guarantee the T° change (cooling in very short time - max 3') weighing heavily on the crystallisation typology of the system.

8. The system, for vibrating unit is able to process from 0 to 250 litres/h.

9. The system can work in continuous and feed directly the plates of the lyophilisator with frozen product in powder form.

10. The system can process products with applicative fields in food, pharmaceutical, cosmetic, veterinary sectors, phitofarmaco, health sectors, and in flavours, fragrances, enzymes, yeast and lactic and not lactic ferments, and in fertilizers sector.
